# EUROPEAN PATENT SPECIFICATION

(11) **EP 4 327 720 B1**
(45) Date of publication and mention of the grant of the patent: **14.10.2026**
(21) Application number: 23193658.4
(22) Date of filing: 28.08.2023
(51) Int. Cl.: A61B 1/00, A61B 1/005, G02B 23/24, A61B 1/015, A61B 1/018

(54) **ENDOSCOPE OPERATION PART AND ENDOSCOPE**
ENDOSKOPBETRIEBSTEIL UND ENDOSKOP
PARTIE D'ACTIONNEMENT D'ENDOSCOPE ET ENDOSCOPE

(30) Priority: 26.08.2022 JP 2022135016
(43) Date of publication of application: 28.02.2024
(73) Proprietor: FUJIFILM Corporation, Tokyo 106-8620 (JP)
(72) Inventor: KUWAE, Toshiharu, Kanagawa, 258-8538 (JP); TANAKA, Kunihiko, Kanagawa, 258-8538 (JP)
(74) Representative: Dehns Germany Partnerschaft mbB

(56) References cited:
- CN-A- 108 601 508
- JP-A- 2011 182 846
- JP-A- 2015 165 842
- US-A1- 2020 397 227
- US-A1- 2022 151 463
- US-B2- 10 918 267

## Description

### BACKGROUND OF THE INVENTION

### 1. Field of the Invention

The present invention relates to an endoscope operation part and an endoscope.

### 2. Description of the Related Art

An endoscope generally comprises an insertion part to be inserted into a body, an operation part connected to a proximal end side of the insertion part, and a universal cable connected to the operation part. The operation part is provided with a rotatable bending operation knob. By rotating the bending operation knob, a bending part provided in the insertion part is bent in up, down, left, and right directions. As a result, the insertion part is smoothly inserted into a patient. In addition, a distal end part of the insertion part can be directed to a desired direction in the body, and an observation image can be acquired from a more suitable position by an optical system provided in the distal end part.

In such an endoscope, a plurality of switches are disposed in the operation part, and various functions related to the endoscope can be operated on a hand-held side. For example, JP1988-222730A (JP-S63-222730A) discloses an endoscope comprising an operation button housed in a recessed portion provided in an operation part. JP2007-325747A discloses an endoscope comprising an operation switch that is provided in an operation part and that is configured to swing in a left-right direction about a rotational movement center axis when viewed from a rear side of the operation part. JP 2015-165842 A relates to a zoom endoscope. US 2020/397227 A1 relates to an external mechanism for endoscope and endoscope system. US 2016/357006 A1 relates to an endoscope.

### SUMMARY OF THE INVENTION

In a case of operating the endoscope, an operator places a connection portion of the universal cable near bases of their thumb and index finger and grips the operation part with their ring finger and little finger. In a case of bending the bending part, the thumb is used to operate a bending operation knob and also used to operate switches. Since these operations are frequently performed during the operation, there is a concern about stress felt by the operator in the endoscope disclosed in JP1988-222730A (JP-S63-222730A) or JP2007-325747A, due to the disposition of the switches and their operational directions.

The present invention has been made in view of such circumstances, and an object of the present invention is to provide an endoscope operation part and an endoscope with improved usability of an operation switch.

An endoscope device is provided as defined by claim 1.

### BRIEF DESCRIPTION OF THE DRAWINGS

Fig. 1 is an overall configuration diagram of an endoscope according to an embodiment.
Fig. 2 is a front view of a distal end surface of a distal end part.
Fig. 3 is a schematic view showing a state in which an endoscope operation part is viewed from a side facing a switch disposition surface.
Fig. 4 is a schematic view showing a state in which the endoscope operation part is viewed from a side facing a bending operation knob.
Fig. 5 is a view showing a state in which the endoscope operation part is viewed from a universal cable connection portion side with respect to the switch disposition surface and from an oblique direction.
Fig. 6 is a view showing a state in which a grip portion is gripped by a left hand in Fig. 3.
Fig. 7 is an enlarged view of an operation switch.
Fig. 8 is an exploded enlarged view of the operation switch.
Fig. 9 is a diagram illustrating an example of a function of the operation switch.
Figs. 10A and 10B are views illustrating modification examples of the endoscope operation part.

### DESCRIPTION OF THE PREFERRED EMBODIMENTS

Hereinafter, embodiments of an endoscope operation part and of an endoscope according to the present invention will be described with reference to the accompanying drawings.

### Endoscope Apparatus

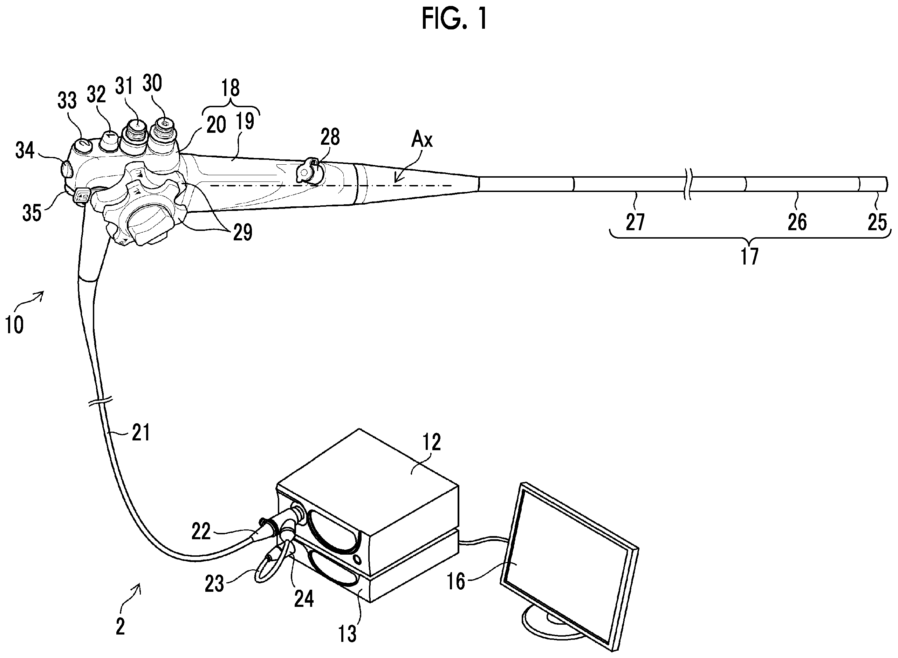

Fig. 1 is a configuration diagram of an endoscope apparatus 2. As shown in Fig. 1, the endoscope apparatus 2 comprises an endoscope 10, a light source device 12, a processor device 13, and a monitor 16.

### Endoscope

The endoscope 10 comprises an elongated insertion part 17 to be inserted into a body, and an endoscope operation part 18 that is consecutively installed on a proximal end side of the insertion part 17.

The endoscope operation part 18 comprises a grip portion 19 and an operation part main body 20, and a distal end side of the grip portion 19 is consecutively installed on the proximal end side of the insertion part 17. The operation part main body 20 is disposed on a proximal end side of the grip portion 19. A universal cable 21 is connected to the operation part main body 20 of the endoscope operation part 18. The universal cable 21 includes a signal cable (not shown), a light guide (not shown), and the like. A light source connector 22 is provided at a distal end of the universal cable 21.

A cable 23 is branched from the light source connector 22, and a processor connector 24 is provided at a distal end of the cable 23. The light source connector 22 is attachably and detachably connected to the light source device 12, and the processor connector 24 is attachably and detachably connected to the processor device 13.

The insertion part 17 is inserted into the lumen (for example, the stomach or the large intestine) of a subject to be examined. The insertion part 17 comprises a distal end part 25, a bending part 26, and a soft part 27 from the distal end side to the proximal end side.

The grip portion 19 is provided with a treatment tool insertion portion 28. Further, the operation part main body 20 is provided with a pair of bending operation knobs 29, an air and water supply button 30, a suction button 31, and switches 32, 33, 34, and 35.

### Distal End Part

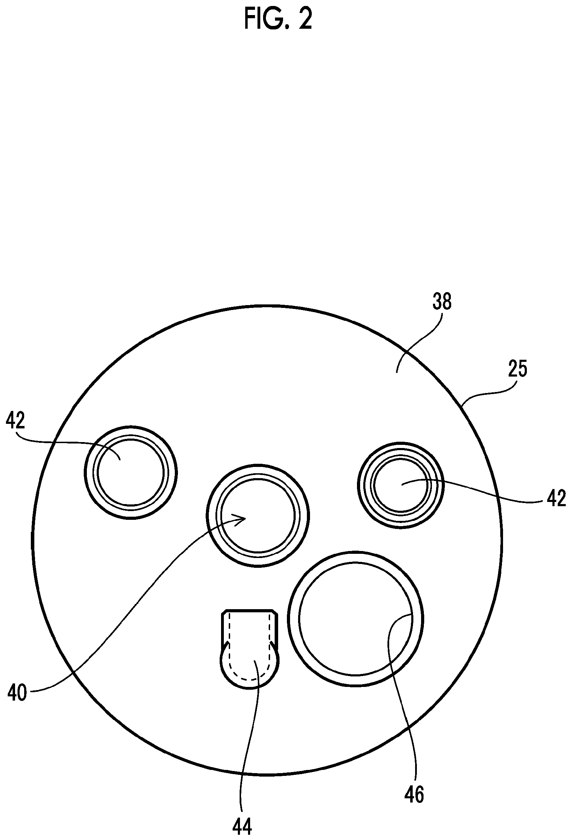

Fig. 2 is a front view of a distal end surface 38 of the distal end part 25. As shown in Fig. 2, on the distal end surface 38 of the distal end part 25, an observation window 40, a pair of illumination windows 42, an air and water supply nozzle 44, and a forceps port 46 are disposed at predetermined positions, respectively. As an example, the observation window 40 is disposed at substantially the center of the distal end surface 38, and the pair of illumination windows 42 are disposed on both sides of the observation window 40. The air and water supply nozzle 44 is disposed at a position where an ejection port faces the observation window 40. The forceps port 46 is disposed in a space surrounded by the observation window 40, one of the illumination windows 42, and the air and water supply nozzle 44.

An image of an observation site illuminated with illumination light from the pair of illumination windows 42 is captured through the observation window 40 by an imaging element (not shown). The captured observation image is input to the processor device 13 via the universal cable 21 or the like, and the observation image is displayed on the monitor 16 by the processor device 13.

### Endoscope Operation Part

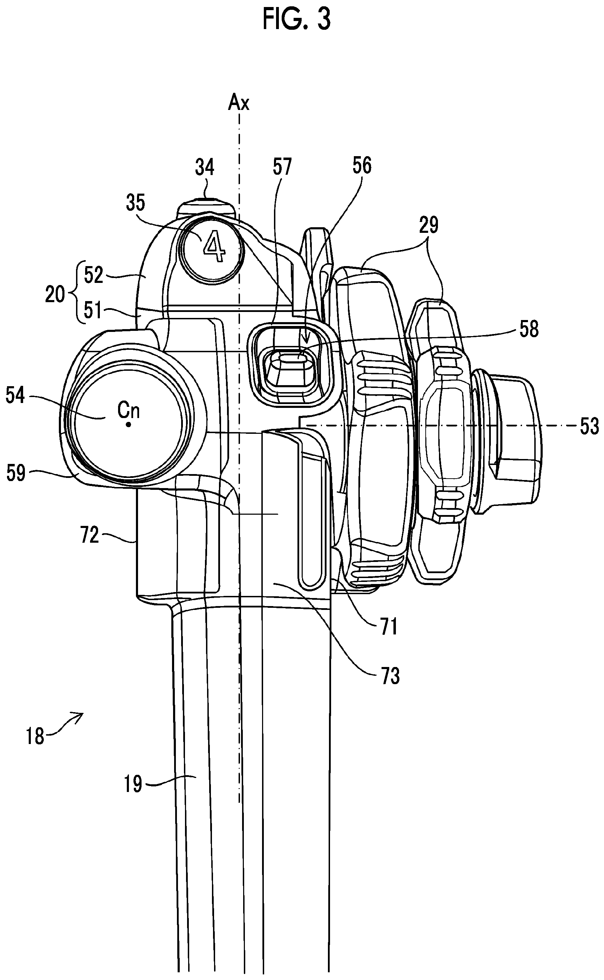

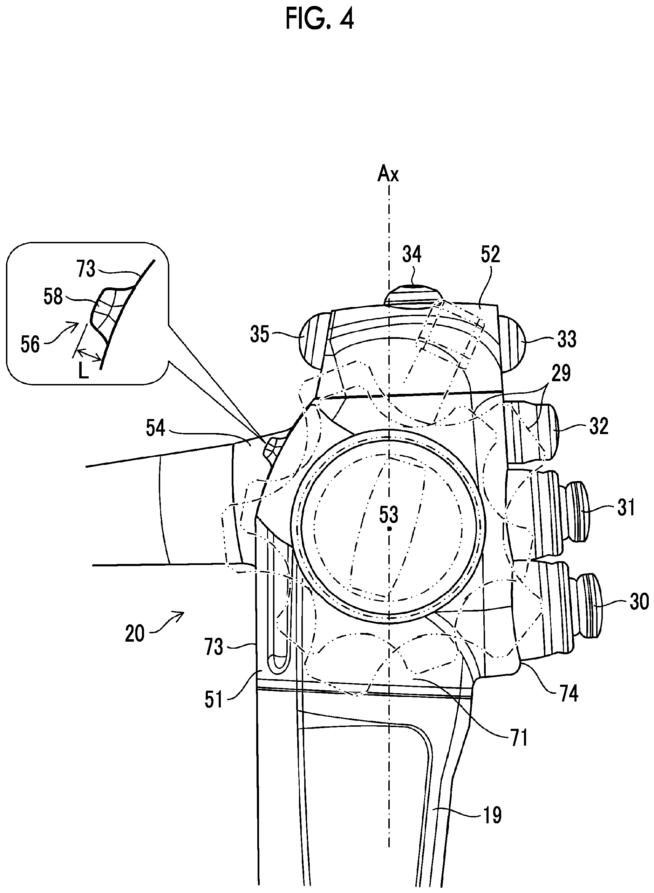

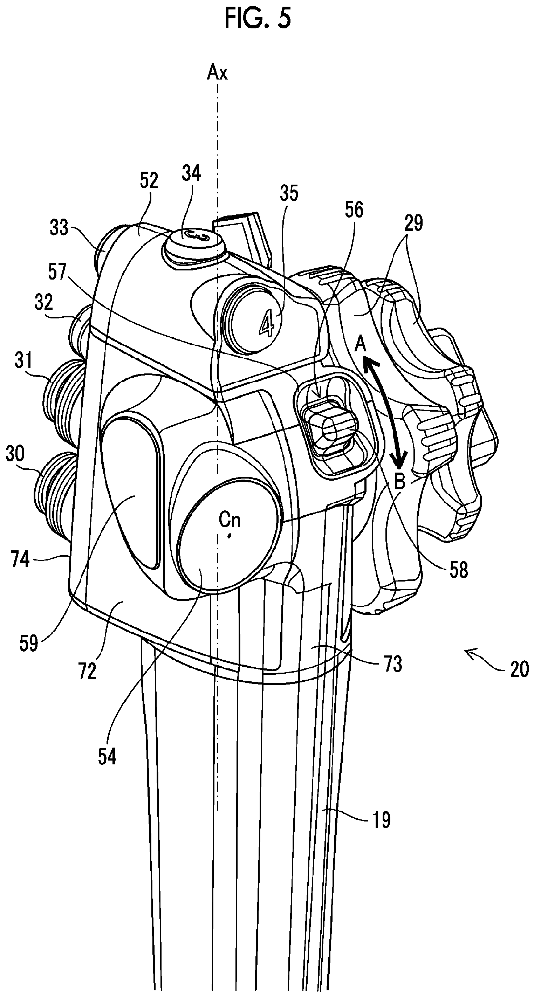

Next, the endoscope operation part 18 according to the embodiment will be described. Fig. 3 is a schematic view showing a state in which the endoscope operation part is viewed from a side facing a switch disposition surface. Fig. 4 is a schematic view showing a state in which the endoscope operation part is viewed from a side facing the bending operation knob. Fig. 5 is a view showing a state in which the endoscope operation part is viewed from a universal cable connection portion side with respect to the switch disposition surface and from an oblique direction.

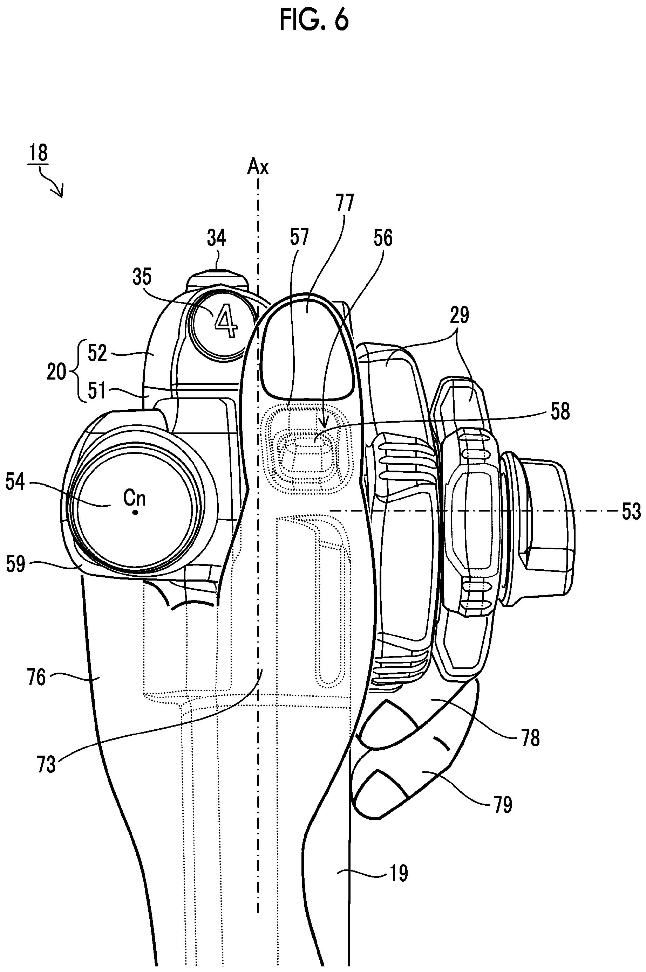

The endoscope operation part 18 is used for an operator to grip and execute an operation of the endoscope apparatus 2. As shown in Figs. 3 to 5, the grip portion 19 provided on one side (downward in Fig. 3) of the endoscope operation part 18 in a longitudinal axis direction Ax and the operation part main body 20 provided on the other side (upward in Fig. 3) opposite to the one side in the longitudinal axis direction Ax are provided. The operation part main body 20 includes a first operation part main body 51 and a second operation part main body 52. The first operation part main body 51 is linked to the grip portion 19 provided downward. The second operation part main body 52 is provided upward of the first operation part main body 51 and is linked to the first operation part main body 51. The terms "upward" and "downward" used here are defined as follows: in a case where the operator grips the endoscope operation part 18 with their left hand and disposes the pair of bending operation knobs 29 on the right side toward the operator, in a case where the endoscope operation part 18 is viewed from a side facing a switch disposition surface 73 (see Figs. 3 and 6), a thumb side of the left hand is defined as "upward" and a little finger side of the left hand is defined as "downward".

As shown in Figs. 3 and 4, the first operation part main body 51 comprises a bending operation knob disposition surface 71 (corresponding to "one side surface" of the embodiment of the present invention). The bending operation knob disposition surface 71 is a side surface facing a direction orthogonal to the longitudinal axis direction Ax and constitutes a surface on which the pair of bending operation knobs 29 are disposed. In Fig. 4, the pair of bending operation knobs 29 are shown by virtual lines. The pair of bending operation knobs 29 are configured to rotate about a rotation axis 53. The operator can bend the bending part 26 and direct the distal end part 25 in a desired direction by rotating the pair of bending operation knobs 29 with their fingers about the rotation axis 53. The rotation axis 53 is substantially orthogonal to the longitudinal axis direction Ax.

As shown in Figs. 3 and 5, the first operation part main body 51 comprises a universal cable connection surface 72 (corresponding to "the other side surface" of the embodiment of the present invention) that is provided opposite to the bending operation knob disposition surface 71. The universal cable connection surface 72 is a side surface facing a direction that is orthogonal to the longitudinal axis direction Ax and that is opposite to the bending operation knob disposition surface 71, and a universal cable connection portion 54 is provided on the universal cable connection surface 72. The proximal end side of the universal cable 21 is connected to the universal cable connection portion 54. The universal cable connection surface 72 is provided with a lead-out part 59 that projects in a direction away from the universal cable connection surface 72, and the universal cable connection portion 54 is provided in the lead-out part 59.

As shown in Fig. 3, the first operation part main body 51 comprises the switch disposition surface 73 located between the bending operation knob disposition surface 71 and the universal cable connection surface 72. An operation switch 56 is provided on the switch disposition surface 73. The operation switch 56 will be described later. As shown in Figs. 4 and 5, the first operation part main body 51 comprises a button disposition surface 74 provided opposite to the switch disposition surface 73.

As shown in Figs. 3 to 5, the first operation part main body 51 is formed in a substantially tubular shape by the bending operation knob disposition surface 71, the universal cable connection surface 72, the switch disposition surface 73, and the button disposition surface 74. The second operation part main body 52 is linked to the bending operation knob disposition surface 71, the universal cable connection surface 72, the switch disposition surface 73, and the button disposition surface 74. The grip portion 19 is linked to the bending operation knob disposition surface 71, the universal cable connection surface 72, the switch disposition surface 73, and the button disposition surface 74 of the first operation part main body 51 and has a substantially tubular shape.

As shown in Figs. 4 and 5, the button disposition surface 74 of the first operation part main body 51 includes the air and water supply button 30, the suction button 31, and the switch 32 disposed in this order from downward to upward along the longitudinal axis direction Ax. The switches 33, 34, and 35 are disposed in the second operation part main body 52 from a button disposition surface 74 side toward a switch disposition surface 73 side in this order. The switch 33 is disposed on a surface that is provided in the second operation part main body 52 and that is continuous with the button disposition surface 74. The switch 34 is disposed most upward on the second operation part main body 52 in the longitudinal axis direction Ax. The switch 35 is disposed on a surface that is provided in the second operation part main body 52 and that is continuous with the switch disposition surface 73.

Each of the switches 32, 33, 34, and 35 can be arbitrarily assigned by the operator for operations of the endoscope apparatus 2, such as freezing the observation image, capturing (releasing) the observation image, and switching special light observation, for example. The switches 32, 33, 34, and 35 include known operation members, such as a button type and a lever type, for example. In a state in which the switches 32, 33, 34, and 35 are not operated, it is preferable that the switches 32, 33, 34, and 35 are each a switch of a type that returns, such as a tact switch, for example.

Next, the operation switch 56 will be described with reference to Figs. 3 to 6. Fig. 6 is a view showing a state in which the grip portion is gripped by the left hand in Fig. 3.

As shown in Fig. 6, in a case where the endoscope operation part 18 is gripped, the operator places the lead-out part 59, on which the universal cable connection portion 54 is formed, near bases of a thumb 77 and of an index finger, and the grip portion 19 is gripped with a ring finger 78 and a little finger 79.

In a case of operating the endoscope 10, the operator operates the bending operation knob 29 with the thumb 77, a middle finger (not shown), the ring finger 78, and the index finger in order to bend the bending part 26 and also operates the switch 35 and the operation switch 56 with the thumb 77. Since this operation is performed frequently during the operation, it is required for the endoscope 10 to improve the usability for the operator to operate the operation switch 56 without stress.

As shown in Figs. 3, 5, and 6, the operation switch 56 is provided on the switch disposition surface 73 of the first operation part main body 51 and is housed in a housing recess 57 provided on the switch disposition surface 73. As shown in Fig. 6, the operation switch 56 is provided within a range reachable by the thumb 77 of a left hand 76 of the operator who grips the grip portion 19.

As shown in Fig. 5, the operation switch 56 is configured to swing in the longitudinal axis direction Ax, which means it can swing in directions substantially parallel to the longitudinal axis direction Ax as indicated by arrows, namely upward A and downward B, which is an opposite direction to upward A. The operator swings the operation switch 56 upward A or downward B with the thumb 77, whereby the endoscope apparatus 2 can exert the assigned specific function.

In the embodiment, the operation switch 56 is made swingable in the longitudinal axis direction Ax (swingable in an up-down direction), which makes it possible to easily operate the operation switch 56 with the thumb 77 and makes it easier for the operator to intuitively grasp the operation direction. It is possible to improve usability of the operation switch 56.

In the embodiment, the operation switch 56 is housed in the housing recess 57. Therefore, as shown in Fig. 4, in a state in which the operation switch 56 is not operated, it is possible to reduce a distance L from the switch disposition surface 73 to a top portion 58 of the operation switch 56, and it is also possible to dispose the top portion 58 below the switch disposition surface 73, that is, not to expose the top portion 58 to the outside. By reducing the distance L, as shown in Fig. 6, the operation switch 56 can be restrained from interfering with the operator's thumb 77 in a case where the operator operates the pair of bending operation knobs 29 with the fingers or a case where the operator moves the thumb 77 between the switch 35 and the pair of bending operation knobs 29. By avoiding the interference, the usability of the operation switch 56 can be improved.

The surface that is provided in the second operation part main body 52 and is continuous with the switch disposition surface 73 corresponds to an example of another switch disposition surface disposed on the other side with respect to the switch disposition surface 73 of the embodiment of the present invention, and the switch 35 corresponds to another switch of the embodiment of the present invention.

Since the operation switch 56 is housed in the housing recess 57, the distance L can be reduced. On the other hand, in a case where the distance L between the top portion 58 and the switch disposition surface 73 is reduced, a contact area (finger grip) between the operator's finger and the operation switch 56 decreases, which may lead to a decrease in operability. However, as shown in Fig. 5, since the operation switch 56 is housed in the housing recess 57, a gap is defined between the top portion 58 and a peripheral edge of the housing recess 57, particularly upward and downward from the top portion 58. As a result, the contact area (finger grip) between the operator's thumb 77 and the operation switch 56 can be secured, and the operability of the operation switch 56 can be ensured.

As shown in Figs. 3, 5, and 6, in a state in which the operation switch 56 is not operated, the position of the top portion 58 of the operation switch 56 in the longitudinal axis direction Ax is located upward of a center position Cn of the universal cable connection portion 54.

As described above, in a case where the lead-out part 59 is placed near the bases of the thumb 77 and of the index finger when the endoscope operation part 18 is gripped, a first joint part of the operator's thumb 77 is located upward of the center position Cn of the universal cable connection portion 54 when the thumb 77 is aligned with the longitudinal axis direction Ax. In a case where the operation switch 56 is located upward of the center position Cn, the operator can easily operate the operation switch 56 with the thumb 77 without re-gripping the grip portion 19. Since the operation switch 56 is located upward of the center position Cn, it is easier to operate the operation switch 56, and the usability of the operation switch 56 can be improved.

In the embodiment, the housing recess 57 has a rectangular shape, and a side (upper side) of the peripheral edge of the housing recess 57, which is located upward, is located upward of the universal cable connection portion 54, and a side (lower side) of the peripheral edge of the housing recess 57, which is located downward, is located in the universal cable connection portion 54. The position of the housing recess 57 is appropriately determined according to a position where the top portion 58 of the operation switch 56 is disposed.

### Preferred embodiments

Next, preferred embodiments of the operation switch 56 will be described. As a first embodiment, as shown in Fig. 4, in the operation switch 56, it is preferable that the top portion 58 of the operation switch 56 is exposed to the outside of the housing recess 57 by a certain distance L in a state in which the operation switch 56 is not operated. It is also possible not to expose the top portion 58 of the operation switch 56 to the outside of the housing recess 57, but in a case where the top portion 58 is exposed to the outside of the housing recess 57, the operator can easily touch the operation switch 56 with the thumb 77, and the operation switch 56 can be more reliably operated. In a case where a portion of the operation switch 56, which is housed in the housing recess 57, is denoted by a distance L1, the relationship between the distance L and the distance L1 is distance L:distance L1 = 1:3 to 1:1. These values are determined in consideration of the prevention of interference of the operation switch 56 and the operability of the operation switch 56.

Next, as a second embodiment, as shown in Figs. 3 and 6, it is preferable that the position of the top portion 58 of the operation switch 56 in the longitudinal axis direction Ax is located upward of a position where the rotation axis 53 of the bending operation knob 29 is provided, in a state in which the operation switch 56 is not operated. As shown in Fig. 6, in a case where the lead-out part 59 is placed near the bases of the thumb 77 and of the index finger when the endoscope operation part 18 is gripped, the first joint part of the thumb 77 is located upward of the rotation axis 53 when the thumb 77 is aligned with the longitudinal axis direction Ax. In a case where the operation switch 56 is located upward of the position where the rotation axis 53 is provided, the operator can easily operate the operation switch 56 with the thumb 77 without re-gripping the grip portion 19.

As shown in Figs. 3 and 6, according to the claimed invention the operation switch 56 is disposed closer to the bending operation knob 29 than to the universal cable connection portion 54. As shown in Fig. 6, the operator can smoothly move the thumb 77 from the bending operation knob 29 to the operation switch 56 or from the operation switch 56 to the bending operation knob 29.

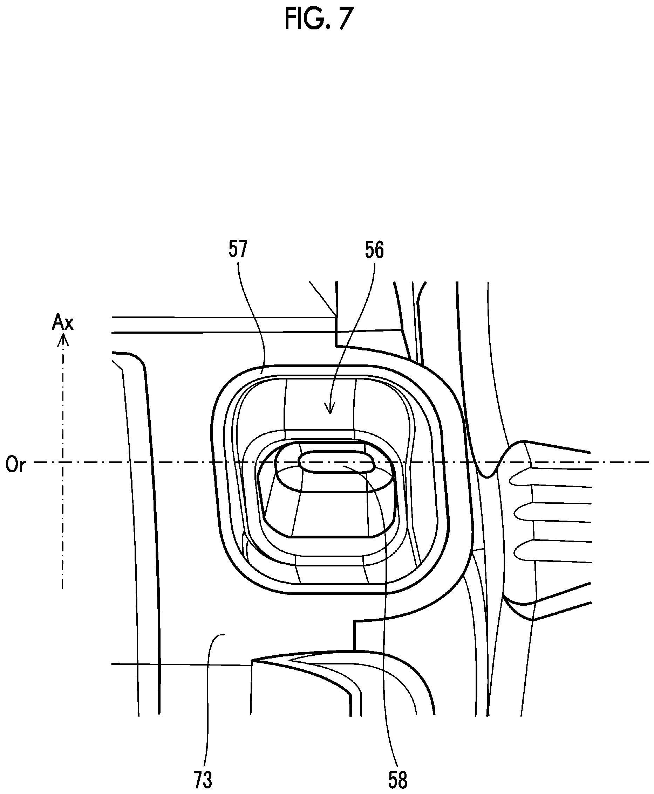

Next, a fourth embodiment will be described with reference to Fig. 7. Fig. 7 is an enlarged view of the operation switch 56 shown in Figs. 3, 5, and 6. As shown in Fig. 7, the operation switch 56 housed in the housing recess 57 comprises the top portion 58. As the fourth embodiment, it is preferable that the top portion 58 is configured to have an elongated shape extending in an orthogonal direction or orthogonal to the longitudinal axis direction Ax in a case where the endoscope operation part 18 is viewed from a side facing the switch disposition surface 73. The top portion 58 is formed in an elongated shape, which makes it easier for the operation switch 56 to be moved in the longitudinal axis direction Ax. In addition, it is easier for the operator to visually recognize a movement direction (up-down direction). The elongated shape of the top portion 58 may be a rectangular shape, a diamond shape, an elliptical shape, or a race track shape (an oval shape). The race track shape is a shape that has a pair of straight portions, which are parallel to each other and which have the same length, and an arc portion which connects end portions of the straight portions. Note that the shape of the top portion 58 is not limited to these shapes as long as the operation switch 56 can move in the longitudinal axis direction Ax. The shape of the top portion 58 may be, for example, a square, a circle, or a triangle.

### Operation Switch

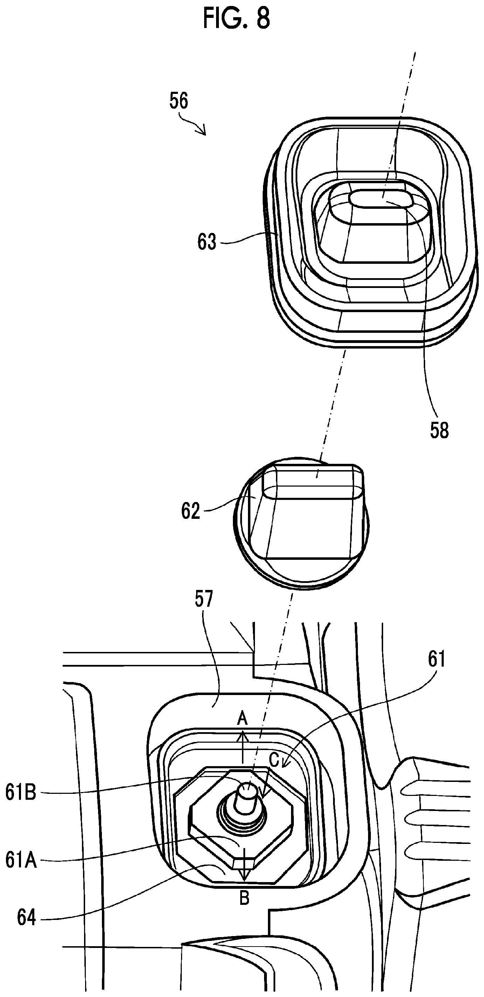

Next, an example of the structure of the operation switch 56 will be described. Fig. 8 is an exploded enlarged view of the operation switch 56. As shown in Fig. 8, the operation switch 56 includes a joystick type switch 61, an intermediate member 62, and a cover 63.

The joystick type switch 61 is electrically connected to a circuit board 64 provided in the housing recess 57. The joystick type switch 61 comprises a base 61A and a stem 61B that projects in a direction orthogonal to an upper surface of the base 61A. The stem 61B is configured to move upward A along the longitudinal axis direction Ax, downward B, that is, in the opposite direction to upward A, and in a pushing direction C orthogonal to the base 61A. That is, the joystick type switch 61 can be pushed in as well as operated in the up-down direction. The joystick type switch 61 comprises, for example, a biasing member inside and is configured to automatically return the stem 61B to a home position in a state in which the joystick type switch 61 is not operated. In a case where the stem 61B is operated upward A, downward B, or in the pushing direction C, the circuit is closed, and the functions assigned to the respective operations are executed in the endoscope apparatus 2.

The intermediate member 62 is attached to the stem 61B. A rod-shaped member (not shown) that comes into contact with an upper surface of the stem 61B is provided inside the intermediate member 62. A pressing force of the operator's thumb 77 is transmitted to the stem 61B of the joystick type switch 61 via the intermediate member 62.

The cover 63 made of the elastic member is attached to the housing recess 57. The top portion 58 of the operation switch 56 is provided as a part of the cover 63. Since the cover 63 is an elastic member, the cover 63 is deformed following the operation of the operator's thumb 77.

In the embodiment, the operation of the joystick type switch 61 is performed via the cover 63. The joystick type switch 61 is not exposed to the outside of the cover 63. As a result, by attaching the cover 63 to the housing recess 57 without using a member such as an O-ring, watertightness with respect to the endoscope operation part 18 can be ensured.

As the operation switch 56, the joystick type switch has been described as an example, but the present invention is not limited thereto, and other switches can be applied.

### Function of Operation Switch

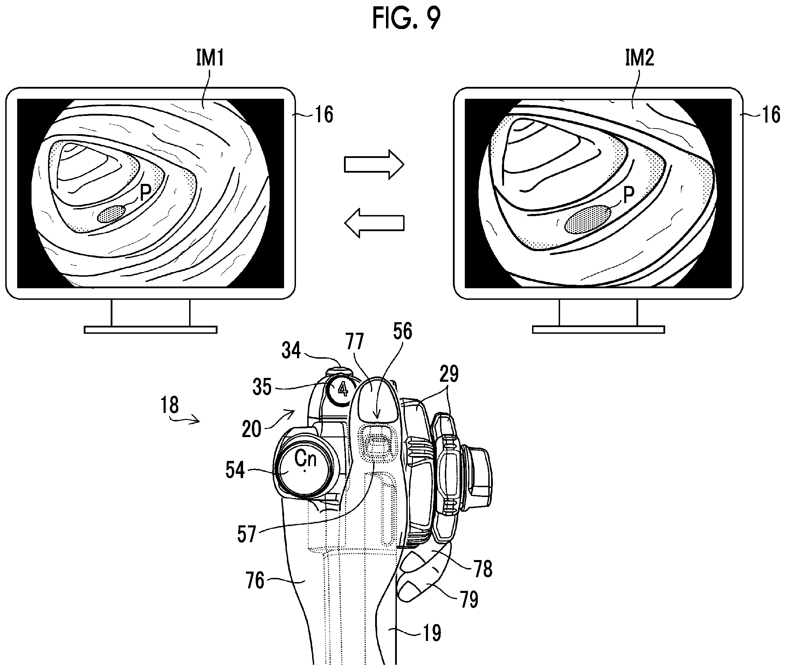

Next, an example of the function assigned to the operation switch 56 will be described with reference to Fig. 9. As shown in Fig. 9, it is preferable that the operation switch 56 is assigned as a zoom operation switch for performing an operation to change the display magnification of the observation image acquired by the endoscope 10.

The operator inserts the insertion part 17 of the endoscope 10 into the subject to be examined, observes the inside of the lumen through the observation window 40 of the distal end part 25, and acquires the observation image. An acquired observation image IM1 is displayed on the monitor 16. A lesion P is shown in the observation image IM1. The operator can, for example, zoom in on a specific location of the observation image IM1 and display an observation image IM2 with an increased display magnification on the monitor 16 by operating the operation switch 56 upward or downward. The lesion P is shown in an enlarged manner in the observation image IM2. In addition, the operator can display the observation image IM1 with a reduced display magnification on the monitor 16 by operating the operation switch 56 downward or upward, which is a direction opposite to the direction in which the operation switch 56 is moved to increase the display magnification, and by zooming out on the observation image IM2. The change in the display magnification of the observation image may be an electronic change in magnification or an optical change in magnification. The function assigned to the operation switch 56 is not limited to the zoom operation.

### Modification Examples

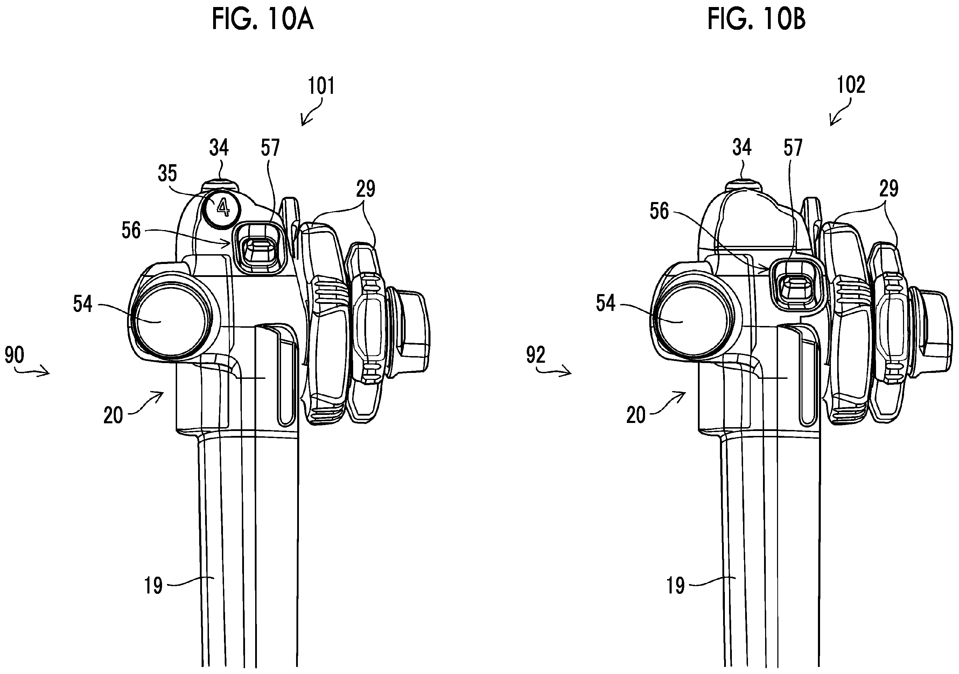

Next, modification examples of the endoscope operation part 18 will be described. Fig. 10A is a view showing a first modification example of the endoscope operation part. Fig. 10A shows a state in which an endoscope operation part 90 is viewed from the side facing the switch disposition surface 73, as in Fig. 3. As shown in Fig. 10A, the position of the operation switch 56 is different between the endoscope operation part 90 of the first modification example and the endoscope operation part 18. As compared with the endoscope operation part 18 with regard to the operation switch 56, the operation switch 56 is disposed closer to the switch 35 in the endoscope operation part 90. In the first modification example, a lower side of the peripheral edge of the housing recess 57 is located upward of the universal cable connection portion 54. In a case where the operation switch 56 is disposed at a position shown in Fig. 10A, the usability of the operation switch 56 can be improved as in the endoscope operation part 18.

Fig. 10B is a view showing a second modification example of the endoscope operation part. Fig. 10B shows a state in which an endoscope operation part 92 is viewed from the side facing the switch disposition surface 73, as in Fig. 3. The presence or absence of the switch 35 is different between the endoscope operation part 92 of the second modification example and the endoscope operation part 18. As compared with the endoscope operation part 18, the endoscope operation part 92 is not provided with the switch 35. Even in a case where the switch 35 is not provided, in a case where the operation switch 56 is disposed at a position shown in Fig. 10B, the usability of the operation switch 56 can be improved as in the endoscope operation part 18.

Although the endoscope operation part and the endoscope according to the embodiment have been described above, some improvements or modifications may be made to the present invention without departing from the gist of the present invention.

### Explanation of References

2: endoscope apparatus
10: endoscope
12: light source device
13: processor device
16: monitor
17: insertion part
18: endoscope operation part
19: grip portion
20: operation part main body
21: universal cable
22: light source connector
23: cable
24: processor connector
25: distal end part
26: bending part
27: soft part
28: treatment tool insertion portion
29: bending operation knob
30: air and water supply button
31: suction button
32: switch
33: switch
34: switch
35: switch
38: distal end surface
40: observation window
42: illumination window
44: air and water supply nozzle
46: forceps port
51: first operation part main body
52: second operation part main body
53: rotation axis
54: universal cable connection portion
56: operation switch
57: housing recess
58: top portion
59: lead-out part
61: joystick type switch
61A: base
61B: stem
62: intermediate member
63: cover
64: circuit board
71: bending operation knob disposition surface
72: universal cable connection surface
73: switch disposition surface
74: button disposition surface
76: left hand
77: thumb
78: ring finger
79: little finger
90: endoscope operation part
92: endoscope operation part
Ax: longitudinal axis direction
A: upward
B: downward
C: pushing direction
Cn: center position
IM1: observation image
IM2: observation image
L: distance
Or: orthogonal direction
P: lesion

## Claims

1. An endoscope operation part (18) comprising:
a grip portion (19) that is provided on one side of the endoscope operation part in a longitudinal axis direction;
an operation part main body (20) that is provided on the other side which is opposite to the one side in the longitudinal axis direction;
a bending operation knob (29) that is disposed on one side surface of the operation part main body;
a universal cable connection portion (54) that is provided on the other side surface of the operation part main body (20), the other side surface being provided opposite to the one side surface; and
an operation switch (56) that is provided within a range reachable by a thumb of an operator who grips the grip portion and that is housed in a housing recess (57) provided on a switch disposition surface (73) which is located in the operation part main body (20) and between the one side surface and the other side surface, the operation switch (56) being configured to swing in the longitudinal axis direction,
wherein, in a case where the endoscope operation part is viewed from a side facing the switch disposition surface (73), a position of a top portion (58) of the operation switch (56) in the longitudinal axis direction is located on the other side with respect to a center position of the universal cable connection portion (54) in a state in which the operation switch (56) is not operated,
wherein the operation switch (56) is disposed closer to the bending operation knob (29) than to the universal cable connection portion (54), and
a first distance (L) is from the switch disposition surface (73) to the top portion (58) of the operation switch (56), and
a second distance (L1) is a length of a portion of the operation switch (56), which is housed in the housing recess,
**characterized in that** the
housing recess (57) has a shape in which an outer opening of the housing recess (57) is wider than an inner bottom of the housing recess (57), and
a ratio (L:L1) of the first distance (L) and the second distance (L1) is between 1:3 and 1:1.

2. The endoscope operation part (18) according to claim 1,
wherein the top portion (58) of the operation switch (56) is exposed to an outside of the housing recess in a state in which the operation switch (56) is not operated.

3. The endoscope operation part (18) according to claim 1 or 2,
wherein, in a case where the endoscope operation part is viewed from the side facing the switch disposition surface (73), the position of the top portion (58) of the operation switch (56) in the longitudinal axis direction is located on the other side with respect to a position where a rotation axis of the bending operation knob (29) is provided, in a state in which the operation switch (56) is not operated.

4. The endoscope operation part (18) according to any one of claims 1 to 3,
wherein the top portion (58) of the operation switch (56) is configured to have an elongated shape extending in a direction orthogonal to the longitudinal axis direction.

5. The endoscope operation part (18) according to any one of claims 1 to 4,
wherein the operation part main body (20) includes another switch provided on another switch disposition surface (73) disposed on the other side with respect to the switch disposition surface (73).

6. The endoscope operation part (18) according to any one of claims 1 to 5,
wherein the operation switch is a zoom operation switch (56) for performing an operation to change a display magnification of an observation image acquired by an endoscope.

7. The endoscope operation part (18) according to any one of claims 1 to 6,
wherein the operation switch (56) is configured to be pushed in.

8. An endoscope comprising:
the endoscope operation part (18) according to any one of claims 1 to 7;
an insertion part (17) that is connected to the one side of the grip portion and is to be inserted into a subject to be examined; and
a universal cable (21) that is connected to the universal cable connection portion (54).

## Patentansprüche

1. Endoskop-Betätigungsteil (18), umfassend:
einen Griffabschnitt (19), der auf einer Seite des Endoskop-Betätigungsteils in einer Längsachsenrichtung vorgesehen ist;
einen Betätigungsteil-Hauptkörper (20), der auf der anderen Seite, die der einen Seite in der Längsachsenrichtung gegenüberliegt, vorgesehen ist;
einen Biegebetätigungsknopf (29), der auf einer Seitenoberfläche des Betätigungsteil-Hauptkörpers angeordnet ist;
einen Universalkabel-Anschlussabschnitt (54), der auf der anderen Seitenoberfläche des Betätigungsteil-Hauptkörpers (20) vorgesehen ist, wobei die andere Seitenoberfläche der einen Seitenoberfläche gegenüberliegend vorgesehen ist; und
einen Betätigungsschalter (56), der innerhalb eines Bereichs, der von einem Daumen eines Bedieners, der den Griffabschnitt greift, erreichbar ist, vorgesehen ist, und der in einer Aufnahmevertiefung (57), die auf einer Schalteranordnungsfläche (73), die sich in dem Betätigungsteil-Hauptkörper (20) und zwischen der einen Seitenoberfläche und
der anderen Seitenoberfläche befindet, vorgesehen ist, aufgenommen ist, wobei der Betätigungsschalter (56) so konfiguriert ist, dass er in der Längsachsenrichtung schwingt,
wobei in einem Fall, in dem der Endoskop-Betätigungsteil von einer Seite, die der Schalteranordnungsfläche (73) zugewandt ist, betrachtet wird, eine Position eines Oberabschnitts (58) des Betätigungsschalters (56) in der Längsachsenrichtung sich auf der anderen Seite in Bezug auf eine Mittelposition des Universalkabel-Anschlussabschnitts (54) in einem Zustand, in dem der Betätigungsschalter (56) nicht betätigt wird, befindet,
wobei der Betätigungsschalter (56) näher an dem Biegebetätigungsknopf (29) als an dem Universalkabel-Anschlussabschnitt (54) angeordnet ist, und
ein erster Abstand (L) von der Schalteranordnungsfläche (73) zu dem Oberabschnitt (58) des Betätigungsschalters (56) ist, und
ein zweiter Abstand (L1) eine Länge eines Abschnitts des Betätigungsschalters (56) ist, der in der Aufnahmevertiefung aufgenommen ist,
**dadurch gekennzeichnet, dass** die Aufnahmevertiefung (57) eine Form aufweist, bei der eine Außenöffnung der Aufnahmevertiefung (57) breiter als ein Innenboden der Aufnahmevertiefung (57) ist, und
ein Verhältnis (L:L1) des ersten Abstands (L) und des zweiten Abstands (L1) zwischen 1:3 und 1:1 liegt.

2. Endoskop-Betätigungsteil (18) nach Anspruch 1,
wobei der Oberabschnitt (58) des Betätigungsschalters (56) in einem Zustand, in dem der Betätigungsschalter (56) nicht betätigt wird, zu einer Außenseite der Aufnahmevertiefung freiliegt.

3. Endoskop-Betätigungsteil (18) nach Anspruch 1 oder 2,
wobei in einem Fall, in dem der Endoskop-Betätigungsteil von der Seite, die der Schalteranordnungsfläche (73) zugewandt ist, betrachtet wird, die Position des Oberabschnitts (58) des Betätigungsschalters (56) in der Längsachsenrichtung sich auf der anderen Seite in Bezug auf eine Position, an der eine Drehachse des Biegebetätigungsknopfs (29) vorgesehen ist, in einem Zustand, in dem der Betätigungsschalter (56) nicht betätigt wird, befindet.

4. Endoskop-Betätigungsteil (18) nach einem der Ansprüche 1 bis 3,
wobei der Oberabschnitt (58) des Betätigungsschalters (56) so konfiguriert ist, dass er eine längliche Form, die sich in einer Richtung senkrecht zu der Längsachsenrichtung erstreckt, aufweist.

5. Endoskop-Betätigungsteil (18) nach einem der Ansprüche 1 bis 4,
wobei der Betätigungsteil-Hauptkörper (20) einen anderen Schalter, der auf einer anderen Schalteranordnungsfläche (73), die auf der anderen Seite in Bezug auf die Schalteranordnungsfläche (73) angeordnet ist, vorgesehen ist, enthält.

6. Endoskop-Betätigungsteil (18) nach einem der Ansprüche 1 bis 5,
wobei der Betätigungsschalter ein Zoom-Betätigungsschalter (56) zum Durchführen einer Bedienung zum Ändern einer Anzeigevergrößerung eines durch ein Endoskop erfassten Beobachtungsbildes ist.

7. Endoskop-Betätigungsteil (18) nach einem der Ansprüche 1 bis 6,
wobei der Betätigungsschalter (56) so konfiguriert ist, dass er eingedrückt wird.

8. Endoskop, umfassend:
den Endoskop-Betätigungsteil (18) nach einem der Ansprüche 1 bis 7;
einen Einführteil (17), der mit der einen Seite des Griffabschnitts verbunden ist und in eine zu untersuchende Untersuchungsperson einzuführen ist; und
ein Universalkabel (21), das mit dem Universalkabel-Anschlussabschnitt (54) verbunden ist.

## Revendications

1. Partie de commande d'endoscope (18) comprenant :
une portion de préhension (19) qui est prévue sur un côté de la partie de commande d'endoscope dans une direction d'axe longitudinal ;
un corps principal de partie de commande (20) qui est prévu sur l'autre côté qui est opposé à l'un côté dans la direction d'axe longitudinal ;
un bouton de commande de courbure (29) qui est disposé sur une surface latérale du corps principal de partie de commande ;
une portion de connexion de câble universel (54) qui est prévue sur l'autre surface latérale du corps principal de partie de commande (20), l'autre surface latérale étant prévue opposée à l'une surface latérale ; et
un interrupteur de commande (56) qui est prévu dans une zone accessible par un pouce d'un opérateur qui saisit la portion de préhension et qui est logé dans un évidement de logement (57) prévu sur une surface de disposition d'interrupteur (73) qui est située dans le corps principal de partie de commande (20) et entre l'une surface latérale et
l'autre surface latérale, l'interrupteur de commande (56) étant configuré pour pivoter dans la direction d'axe longitudinal,
dans laquelle, dans un cas où la partie de commande d'endoscope est vue depuis un côté faisant face à la surface de disposition d'interrupteur (73), une position d'une partie supérieure (58) de l'interrupteur de commande (56) dans la direction d'axe longitudinal est située de l'autre côté par rapport à une position centrale de la portion de connexion de câble universel (54) dans un état où l'interrupteur de commande (56) n'est pas actionné,
dans laquelle l'interrupteur de commande (56) est disposé plus près du bouton de commande de courbure (29) que de la portion de connexion de câble universel (54), et
une première distance (L) s'étend entre la surface de disposition d'interrupteur (73) et la partie supérieure (58) de l'interrupteur de commande (56), et
une deuxième distance (L1) est une longueur d'une portion de l'interrupteur de commande (56), qui est logée dans l'évidement de logement,
**caractérisée en ce que** l'évidement de logement (57) présente une forme dans laquelle une ouverture extérieure de l'évidement de logement (57) est plus large qu'un fond intérieur de l'évidement de logement (57), et
un rapport (L:L1) entre la première distance (L) et la deuxième distance (L1) est compris entre 1:3 et 1:1.

2. Partie de commande d'endoscope (18) selon la revendication 1,
dans laquelle la partie supérieure (58) de l'interrupteur de commande (56) est exposée à l'extérieur de l'évidement de logement dans un état où l'interrupteur de commande (56) n'est pas actionné.

3. Partie de commande d'endoscope (18) selon la revendication 1 ou la revendication 2, dans laquelle, dans un cas où la partie de commande d'endoscope est vue depuis le côté faisant face à la surface de disposition d'interrupteur (73), la position de la partie supérieure (58) de l'interrupteur de commande (56) dans la direction d'axe longitudinal est située de l'autre côté par rapport à une position où un axe de rotation du bouton de commande de courbure (29) est prévu, dans un état où l'interrupteur de commande (56) n'est pas actionné.

4. Partie de commande d'endoscope (18) selon l'une quelconque des revendications 1 à 3, dans laquelle la partie supérieure (58) de l'interrupteur de commande (56) est configurée pour présenter une forme allongée s'étendant dans une direction orthogonale à la direction d'axe longitudinal.

5. Partie de commande d'endoscope (18) selon l'une quelconque des revendications 1 à 4, dans laquelle le corps principal de partie de commande (20) inclut un autre interrupteur prévu sur une autre surface de disposition d'interrupteur (73) disposée de l'autre côté par rapport à la surface de disposition d'interrupteur (73).

6. Partie de commande d'endoscope (18) selon l'une quelconque des revendications 1 à 5, dans laquelle l'interrupteur de commande est un interrupteur de commande de zoom (56) destiné à effectuer une opération de modification d'un grossissement d'affichage d'une image d'observation acquise par un endoscope.

7. Partie de commande d'endoscope (18) selon l'une quelconque des revendications 1 à 6, dans laquelle l'interrupteur de commande (56) est configuré pour être enfoncé.

8. Endoscope comprenant :
la partie de commande d'endoscope (18) selon l'une quelconque des revendications 1 à 7 ;
une partie d'insertion (17) qui est connectée à l'un côté de la portion de préhension et qui est destinée à être insérée dans un sujet à examiner ; et
un câble universel (21) qui est connecté à la portion de connexion de câble universel (54).
